**Europäisches Patentamt**

(19) **European Patent Office**

**Office européen des brevets**

(11) Publication number : **0 392 377 B1**

# EUROPEAN PATENT SPECIFICATION

(45) Date of publication of patent specification :
**15.02.95 Bulletin 95/07**

(51) Int. Cl.⁶ : **G01N 33/49, B01D 39/00**

(21) Application number : **90106605.0**

(22) Date of filing : **06.04.90**

(54) **Method and device for the separation of plasma or serum from whole blood.**

(30) Priority : **07.04.89 US 335064**
**27.03.90 US 499864**
**27.03.90 US 499928**

(43) Date of publication of application :
**17.10.90 Bulletin 90/42**

(45) Publication of the grant of the patent :
**15.02.95 Bulletin 95/07**

(84) Designated Contracting States :
**AT BE CH DE ES FR GB IT LI NL**

(56) References cited :
**EP-A- 0 095 057**
**EP-A- 0 133 895**
**EP-A- 0 223 978**
**EP-A- 0 305 803**
**US-A- 4 753 776**

(73) Proprietor : **ABBOTT LABORATORIES**
**CHAD-0377,**
**AP6D/2,**
**One Abbott Park Road**
**Abbott Park, Illinois 60064-3500 (US)**

(72) Inventor : **Aunet, Diane L.**
**138 Washington Park**
**Waukegan, IL 60085 (US)**
Inventor : **Elmore, Kristin D.**
**3784 DeWoody Road**
**Waukegan, IL 60087 (US)**
Inventor : **Georgevich, Gradimir G.**
**813 Dublin**
**Mundelein, IL 60060 (US)**
Inventor : **Oosta, Gary M.**
**5377 Ashwood Lane**
**Gurnee, IL 60031 (US)**
Inventor : **Siegel, Neal A.**
**531 Mallard Lane**
**Deerfield, IL 60015 (US)**
Inventor : **Jeng, Tzyy-Wen**
**311 Appleton Drive**
**Vernon Hills, IL 60061 (US)**
Inventor : **Pry, Terry A.**
**820 Arthur**
**Libertyville, IL 60048 (US)**
Inventor : **Silverman, Cathy R.**
**4369 Finch Court,**
**Apt. 7**
**Gurnee, IL 60031 (US)**

(74) Representative : **Modiano, Guido, Dr.-Ing. et al**
**Modiano & Associati S.r.l.**
**Via Meravigli, 16**
**I-20123 Milano (IT)**

EP 0 392 377 B1

## Description

The present invention relates generally to methods for separating plasma or serum from whole blood. More particularly, the invention relates to devices capable of separating plasma or serum from whole blood comprising a hydrophilic sintered porous material in which at least one red blood cell agglutinating agent has been incorporated. Agglutinated blood cells are removed from whole blood by the sieving action of the matrix of the sintered porous material, and optional additional filter means.

### Background

Modern clinical diagnostic methods are routinely carried out on blood samples. Unfortunately, red blood cells present in whole blood scatter and absorb light thus interfering with assay methodologies which measure either reflected or transmitted light. Other cells may interfere with particular determinations; for example, cholesterol determinations can be affected by cholesterol present in cell membranes. For this reason, many assay methodologies are carried out on plasma or serum which must be separated from a whole blood sample.

Centrifugation is a well-known method in the art by which plasma (before clotting) and serum (after clotting) is separated from whole blood. Stratifying whole blood by centrifugation, however, is time consuming and requires cumbersome laboratory equipment. The use of red blood cell agglutinating agents such as those disclosed in Van Oss, et al., Vox. Sang., vol. 34, pp 351-361 (1978) can be helpful in carrying out centrifugation and other red blood cell separation techniques.

Dojki, et al., U.S. Patent No. 4,464,254, issued 7 August 1984, disclose a piston device capable of isolating serum from an already stratified blood sample. The device consists of a piston head connected to an open-ended sampling tube. The piston head is composed of a one-way valve under which is located a cavity containing a porous plastic filter body. Insertion of the piston head-sampling tube assembly into a test tube containing a stratified sample of blood allows serum to pass through the filter body and valve into the interior of the sampling tube. The volume and purity of the serum which can be separated from the whole blood is contingent upon the completeness of the stratification of the blood.

Vogel, et al., U.S. Patent No. 4,477,575, issued 16 October 1984, disclose a device and a process using the device to separate serum from whole blood by causing whole blood to pass into and through a layer of glass fibers with diameters from 0.2 to 5 $\mu$m and with a density of 0.1 to 0.5 g/cm$^3$. The volume of plasma or serum which can be separated from whole blood by this device is disclosed to be less than 50% of the absorption volume of the glass fiber layer.

Zuk, U.S. Patent No. 4,594,327, issued 10 June 1986, discloses an analytical method wherein a whole blood sample is combined with a red blood cell binding agent and the mixture is then filtered through a solid bibulous element to which is bound at least one specific binding pair member so as to remove the agglutinated red blood cells. The patent discloses anti-red blood cell antibodies, polymeric amino acids, such as polylysine, and lectins, such as wheat germ agglutinin, as suitable red blood cell binding agents for causing the aggregation of red blood cells in whole blood.

Hillman, et al., U.S. Patent No. 4,753,776, issued 28 June 1988, disclose a device and a process using the device to separate serum from whole blood using capillary action to pass whole blood through a glass microfiber filter. The patent discloses an alternative embodiment in which whole blood is passed through a filter to which red blood cell agglutinins have been attached. Rather than retaining the red blood cells, however, the filter disclosed merely retards their flow, eventually allowing their escape.

Trasch, et al., EPO Publication No. 133,895, published March 13, 1985, disclose a red blood cell retaining substrate and a process using the substrate for retaining red blood cells on filters thus allowing the recovery of plasma from whole blood. The red blood cell-retaining substrates of the invention are stated to induce coagulation, but not hemolysis, so that the coagulated corpuscular components can be removed on a filter, while the plasma passes through. The publication discloses alternative embodiments where the retaining substrate is incorporated into the filter or into a pre-filter layer. The publication states that absorptive, porous, liquid-permeable carriers or filters, in the form of paper, fleece, gel or tissues, comprised of cellulose, wool, glass fiber, asbestos, synthetic fibers, polymers or mixtures of the same, can be used as the absorptive materials for the retaining zone.

Most portable techniques for the separation of serum or plasma are limited with respect to speed and serum yield efficiency. Blood separation devices utilizing glass fiber membranes, for example, tend to separate serum at a relatively slow speed and tend to retain significant quantities of serum or plasma in the interstices of the membrane. Accordingly, there exists a desire in the art for improved devices providing rapid and efficient methods for serum and plasma separation.

Another difficulty encountered in the testing of blood samples is that it is generally necessary to measure a precise test sample volume of plasma or serum for use in diagnostic assays. This need for precision is typically met by having a trained technician use a sophisticated pipetting apparatus or by the use of expensive automated instruments. There are also test strip devices which use membrane or paper matrices to collect a plasma sample and transport that sample to a reaction zone on the test strip. Test strip devices, however, typically provide only that sample volume capacity which is needed to transport sample by capillary action through the strip to the reaction zone, and therefore a low level of precision is required. In test strips devices, the plasma recipient member only collects that amount of sample necessary to fill the strip which in turn ends the migration of the scale through the strip because the drawing force which causes sample subject to analysis in a test strip device is limited to that amount which passes through a defined detection zone on the test strip before the strip is filled.

Also, the separation of various reactant components of a mixture from each other so that a chosen reaction occurs only upon their mixing is desirable in clinical and research laboratories. These components may be provided in a dry or a liquid state. It is known that reagents in a liquid state often have a shorter or limited active life as compared to reagents stored in a dried state.

Reagents have been provided in a freeze-dried, so-called lyophilized form in the past. For example, U.S. Patent No. 4,447,526 teaches a carrier matrix for a test strip which is incorporated with reagents either by dipping a carrier matrix sequentially into each of two or more solutions or suspensions of reagents with drying steps in between dippings, or by affixing two or more layers of a carrier material one on top of the other to make a multiphasic carrier matrix for a test strip. Examples of carrier matrices included felt, woven or matted glass fibers, paper and polymeric microcapsules which rupture when contacted with a test sample or liquid. However, this patent does not teach a unitized form of delivering a reagent into a solution.

U. K. Patent Application No. GB2216258A teaches an inert porous matrix which contains a predetermined volume of a chemical reagent which is air-dried and later is recoverable into solution. The patent application teaches that drawbacks of freeze-drying include the problems associated with reconstituting, diluting and producing a measurable required dose of reagent at or near the time of use.

It would be advantageous to provide a porous carrier which retained a freeze-dried reagent which is substantially recoverable into solution in a short period of time. Such a carrier could incorporate two or more reagents which are not reactive with each other. Further, the carrier could contain reagents such as biologically active molecules, compounds and the like which then could be stored in a freeze-dried state over a period of time and be substantially recoverable into solution when desired. In another aspect, the carrier could contain reagents such as biologically active molecules, compounds and the like as well as chemical reagent(s).

## SUMMARY OF THE INVENTION

The present invention relates to improved methods, devices and kits for separating plasma or serum from whole blood. Specifically, the devices of the invention comprise a matrix of hydrophilic sintered porous material in which at least one red blood cell agglutinating agent has been incorporated. The matrix is further characterized by a pore size selected such that individual blood cells will pass through the matrix but wherein agglutinated blood cells will be retained by the matrix. The devices are capable of carrying out a rapid separation of serum or plasma from whole blood while retaining only minimal quantities of serum or plasma within the interstices of the matrix.

According to one aspect of the invention, the device comprises a matrix of hydrophilic sintered porous material in which at least one red blood cell agglutinating agent has been incorporated. A sample of whole blood is applied to an inlet of the matrix and the blood cells within the sample come in contact with the agglutinating agents present in the matrix. The blood cells agglutinate and are entrapped in the interstices near the inlet of the matrix, while substantially blood-cell-free serum or plasma accumulates near an outlet of the matrix. A receiving means, including materials such as filter paper or additional porous matrices, may be incorporated in liquid receiving relationship with the outlet of the matrix. The receiving means functions to wick the substantially blood-cell-free serum or plasma from the outlet of the matrix, thus making the serum or plasma available for analysis or other purposes.

According to an alternative aspect of the invention, a filter means is incorporated in liquid receiving relationship with the outlet of the matrix for improved efficiency and more rapid separation of the blood cells from a sample of whole blood. The filter means may have at least one red blood cell agglutinating agent incorporated therein in order to assist in retaining the blood cells. The methods and devices of the invention can be utilized for the analysis of selected components of blood plasma or serum when the devices comprise the porous matrix of the invention in combination with additional matrices or filter means in which analytical reagents selected for reaction with the selected components may be incorporated.

As indicated previously, removal of red blood cells is of particular interest in visually red assays. Nevertheless, removal of other blood cells is desirable as well, and is to be understood when the term "red blood cell" is employed herein in the context of retention in the matrix or removal of whole blood.

The devices and methods of the present invention can also be utilized for the collection of a predetermined volume of plasma or serum test samples when the devices comprise an additional matrix of sintered porous material that is characterized by a reproducible fluid uptake capacity proportional to the fixed dimensions of said additional matrix, a minimal reactivity with plasma or serum components, and a hydrophilic internal surface, wherein said additional matrix is encased in a housing means whereby an entry port to the matrix is defined. These characteristics enable the additional matrix to collect and retain a predetermined volume of sample for analysis. Optionally, an exit port from the matrix is also defined by the encasement means.

The sintered porous materials used to make the collection matrix include sintered glass, sintered steel, sintered ceramics, sintered plastics and equivalents thereof. A particularly preferred material is polyethylene.

The collection matrix is used in conjunction with a blood separator means of the invention which separates plasma or serum from a whole blood sample. Typically, the matrix is in liquid receiving relationship with the blood separator means, and the matrix thereby collects a predetermined volume of plasma or serum from the blood separator means. The collection matrix can also be used in conjunction with a sample receiver means to which the matrix transfers the predetermined volume of sample for analysis. Alternatively, the analysis can be performed upon the plasma or serum sample in the matrix itself.

Suitable sample receiver means include reaction or detection vessels, such as cuvettes, test tubes, slides and reaction wells. The sample is eluted into the detection vessel by the application of an eluting buffer to the matrix. Other sample receiver means include absorbent solid phase materials having a pore size selected to induce the flow of sample from the matrix into the absorbent by capillary action. The sample receiver means can optionally include one or more analytical reagents which are reconstituted upon the transfer of test sample to the receiver means.

The collection of a serum or plasma sample for analysis is performed by applying a quantity of serum or plasma to the collection matrix and thereby collecting a predetermined volume of plasma or serum.

The device of the present invention may also comprise an improved system for delivering reagents in a freeze-dried unitized form. Specifically, the system comprises a matrix of sintered porous material in which at least one reagent has been incorporated. The matrix is further characterized by a pore size selected such that reagents are retained by the matrix in a dried state but are released from the matrix when contacted with a liquid. The system is capable of delivering reagents to a liquid while retaining only minimum quantities of the reagent within the interstices of the matrix.

The matrix of sintered material in which at least one reagent has been incorporated can be prepared for use by first placing a porous matrix in a container of a reagent solution and allowing it to saturate. After saturation, the porous matrix is frozen and lyophilized by methods known to those of ordinary skill in the art.

## BRIEF DESCRIPTION OF THE FIGURES

Figure 1 is a depiction of a device comprising a porous matrix and a filter paper receiving matrix;

Figure 2 is a depiction of a device comprising a first porous matrix, a second porous matrix, and a filter paper receiving matrix;

Figure 3 is a depiction of a device comprising a porous matrix, and a filter paper receiving matrix with a reagent containing zone;

Figure 4 is a depiction of a device comprising a first porous matrix, a first filter means, a second porous matrix, a second filter means, and a receiving porous matrix; and

Figure 5 is a depiction of a device comprising a porous matrix, a filter means, and a receiving porous matrix.

Figure 6 is a graph of the absorbance spectra of reaction product wherein absorbance readings for glucose standards at 0, 100, 300 and 800 mg/dL as plotted as a function of absorbance v. wavelength, wherein the highest peak was obtained from a glucose standard at 800 mg/dL, the second highest peak was obtained from a glucose standard at 300 mg/dL, the third highest peak was obtained from a glucose standard at 100 mg/dL and a flat line was obtained from a glucose standard at 0 mg/dL.

Figure 7 is a graph of a dose response curve of a glucose assay wherein the mean absorbance values (N=8) at 512, 450 and 560 nm for each glucose standard at 100, 300 and 800 mg/dL were plotted; the curve of wavelength 512 nm is represented by the line drawn between squares, the curve of wavelength 450 nm is represented by the line drawn between circles, and the curve of wavelength 560 nm is represented by the line drawn between diamonds.

Figure 8 is a linear regression curve for triglyceride standards wherein mean absorbance readings at 512 nm were plotted as a function of absorbance v. triglyceride concentration.

## DETAILED DESCRIPTION OF THE INVENTION

The present invention provides improved devices and methods for using those devices to separate plasma or serum from whole blood. The devices of the invention comprise matrices of hydrophilic sintered porous materials in which at least one red blood cell agglutinating agent has been incorporated. The matrix is characterized by a pore size such that individual blood cells will pass through it, but wherein agglutinated cells will be retained by the matrix. The devices are capable of performing rapid separations of serum or plasma from whole blood while retaining only minimal residual quantities of serum or plasma within the interstices of the porous material.

The device of the present invention may also comprise an improved system for containing and delivering reagents. The system comprises a matrix of sintered porous materials in which at least one reagent has been contained. The matrix is characterized by a pore size such that reagents will be retained within it in a freeze-dried state, but will pass through the matrix when a liquid and the matrix are brought into contact with each other.

Among the materials contemplated as being suitable for the matrices used in the devices of the present invention are sintered glass, sintered steel, sintered ceramics, and sintered polymers of plastic, with the preferred material being that known as sintered polyethylene such as that described in British patent 2,186,205. Sintered polyethylene matrices commercially available from Porex, Inc., Fairburn, Georgia or General Polymeric Corp., West Reading, Pennsylvania may be obtained which have a pore size of from about 10 μm to about 70 μm. Such a pore size allows individual red blood cells to pass through the matrix, but retains agglutinated red blood cells within the matrix.

The matrices used in the devices of the present invention are hydrophilic so as to promote the flow of aqueous liquids through them. Commercially available matrices may be either hydrophilic or hydrophobic in nature. Hydrophobic matrices may be rendered hydrophilic by a variety of known methods. Among those methods available are plasma treatment or surfactant treatment of the matrix. Plasma treatment involves exposing the hydrophobic matrix to charged gas (plasma) wherein an electronic charge is imparted to the solid surface rendering the surface wettable. Surfactant treatment involves dipping the hydrophobic matrix in a surfactant and letting it dry. This treatment assists in wetting the surface and interior of the matrix and results in the promotion of aqueous liquid flow through the matrix. It is contemplated that a wide variety of commercially available surfactant materials would be appropriate for use with the present invention. In the assays discussed in the Examples below, commercially available matrices which had been co-molded with surfactant were used and are preferred over matrices dipped in commercially available surfactants. In some of the Examples of the reagent delivery system below, commercially available matrices which had been comolded with surfactant were used and are preferred over matrices dipped in commercially available surfactants. Other examples of the reagent delivery system used commercially available matrices which did not contain surfactant.

In general, surfactants should be selected which are compatible with the reactants or reagents placed within the matrix so as not to interfere with the preferred activity. Additionally, it should be noted that no surfactant should be present in such concentrations as to cause hemolysis of the red blood cells. In addition, care must be exercised to avoid hemodilution of the plasma sample. Hemodilution is the extraction into the plasma of the internal fluid of the red blood cell due to hypertonic conditions.

The incorporation of anti-coagulants into whole blood samples is particularly preferred for promoting the flow of plasma through the devices. Anti-coagulants mixed with the blood before application to the device prevent the blood from clotting. Separation of blood cells from a blood sample treated with anti-coagulants produces plasma. Separation of red blood cells from a clotted blood sample produces serum. It is further contemplated that these anti-coagulants may be incorporated into the matrices to prevent the blood sample from clotting when applied to the device. For example, a drop of blood from a finger stick may be directly applied to the device such that anti-coagulants incorporated within the device come in contact with the blood and prevent the blood from clotting. Alternatively, blood can be collected in a capillary tube previously treated with anticoagulant, and transferred to the device in this manner. Preferred anticoagulant materials include heparin, EDTA and citrate.

According to the invention, red blood cell agglutinating agents are incorporated into the porous matrices. Agglutinating agents are substances which cause individual red blood cells to adhere to one another to form clumps. It is contemplated that the agglutinating agents may be incorporated into a matrix by means such as adsorption, absorption or metallic-organic dye complexes, although it is preferred that at least some of the agglutinating agent be absorbed into the matrix such that it may be solubilized in the presence of a blood sample.

Suitable agglutinating agents include natural and synthetic water soluble polymers including, but not limited to, those discussed in the background. Among the available agglutinins, preferred agglutinins include hexadimethrine bromide, which is available from Aldrich Fine Chemicals as Polybrene®, polylysine, and anti-red

EP 0 392 377 B1

blood cell antibodies. It is believed that positively charged polyelectrolytes, such as Polybrene® and polylysine, aggregate erythrocytes due to charge neutralization, changes in hydration, polymer bridging and osmotic interaction. IgG- or IgM-class antibodies specific for red blood cell antigens cause agglutination by binding to similar antigenic determinants on the surface of two separate erythrocytes which causes the cells to adhere to one another. An additional enhancement of the agglutination process is achieved by incorporating substances such as polyvinyl pyrrolidone (PVP) which apparently function as dielectrics allowing charged cells to approach one another and be crosslinked by antibody and/or other agglutinins.

A high agglutinating agent concentration results in a longer residence time for a blood sample within the matrix and increases the efficiency of agglutination of red blood cells within the matrix. This can have the undesirable effect, however, of trapping a large proportion of the plasma within the matrix. Conversely, lowering the agglutinating agent concentration allows more plasma to be released, but may result in fewer red blood cells within the sample being trapped by the matrix. The length, volume, and porosity of the matrix, as well as the volume of the blood sample to be filtered by the matrix, in addition to the agglutinating agent concentration affect the efficiency of entrapment of red blood cells within the matrix and the amount of plasma eluted by the matrix.

According to a first preferred embodiment of the device of the present invention, the pore size of the matrix is selected in conjunction with the length and volume of the matrix, the volume of blood sample to be treated, and the agglutinating agent's ability to cause the red blood cells to clump together, such that substantially all of the red blood cells present in a whole blood sample become agglutinated and are retained in the matrix. Removal of "substantially all" red blood cells present in a blood sample constitutes the removal of a sufficient amount of the red blood cells from the sample so that a clinical determination of a selected blood analyte may be performed without interference. Preferably, removal of "substantially all" red blood cells present in a blood sample constitutes the removal of at least about 90% of the red blood cells from the sample.

According to one method of utilizing the first preferred embodiment of the device of the present invention, a sample of whole blood is applied to an inlet or first end of the matrix. The blood rapidly passes through the interstices of the matrix, quickly coming in contact with the red blood cell agglutinating agents incorporated therein. These agents promote agglutination of the red blood cells which are then entrapped within the interstices of the matrix. This entrapment of the agglutinated red blood cells within the matrix permits the rapid and efficient separation of plasma or serum from the red blood cells. Additionally, because the matrix retains only a minimal amount of plasma or serum, a large amount of the plasma or serum may be successfully harvested from the whole blood sample. Optionally, a filter means such as filter paper or an additional porous matrix may be placed in liquid receiving relationship with the outlet of the matrix in order to wick the serum or plasma from the matrix.

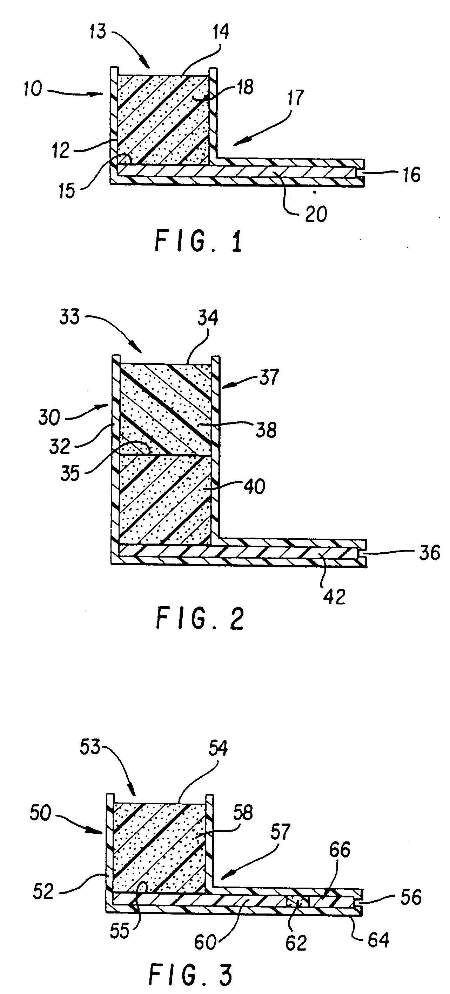

Figures 1-2 are depictions of exemplary devices used to separate plasma from whole blood according to the first embodiment of the present invention. As illustrated in Figure 1, an apparatus (10) comprises a housing (12) which has an entry port (13) and an exit vent (16). Located within the housing (12) is a device (17) comprising a porous polyethylene matrix (18) which contains an agglutinating agent and is molded into a cylindrical shape having the dimensions of 3.5 mm in diameter and 5 mm in height. The exact shape and dimensions are not critical to the invention but affect resident time and efficiency as described herein. Also located within the housing (12) is a paper matrix (20). The matrix (18) has an inlet (14) and an outlet (15) and is in liquid receiving contact with said paper matrix (20). The paper matrix (20) and the matrix (18) may contain the reagents necessary for the analysis of a selected blood analyte.

As illustrated in Figure 2, an apparatus (30) comprises a housing (32) which has an entry port (33) and an exit vent (36). Located within the housing (32) is a device (37) comprising a first porous polyethylene matrix (38). Also located within the housing (32) is a second porous polyethylene matrix (40) in liquid receiving relationship with said first matrix and a paper matrix (42) in liquid receiving relationship with said second matrix. The first matrix (38) contains an agglutinating agent and has an inlet (34) and an outlet (35). The second matrix (40) contains some of the reagents necessary for the determination of a specific blood analyte while the paper matrix (42) contains the other components of the reagent system. It is contemplated that the first matrix (38) may also contain reagents necessary far the analysis of a selected blood analyte. An exemplary dye paper reagent system is described in U.S. Serial No. 204,443 filed June 9, 1988 and incorporated herein by reference.

According to a second preferred embodiment of the device capable of more rapid separation of red blood cells, the pore size of the matrix is selected in conjunction with the length and volume of the matrix, the volume of blood sample to be treated, and the agglutinating agent's ability to cause the red blood cells to clump together, such that less than all the red blood cells present in a whole blood sample become agglutinated and are retained in the matrix. In these cases where it is desirable to select a matrix having a relatively large pore size which provides a high rate of flow, but wherein not all the red blood cells are retained by the matrix, the red blood cells remaining in the plasma or serum are subjected to subsequent filtration steps utilizing second-

6

ary matrices or filters alone, or impregnated with red blood cell agglutinating agents, such that "clear" plasma or serum is produced. The removal of at least 97% of the red blood cells from the sample constitutes "clear" plasma or serum.

Filter paper characterized by a pore size such that agglutinated red blood cells will not pass through it may be used to purify further the serum or plasma. Additionally, this filter paper has agglutinating agents incorporated within it to aid in the retention of the remaining red blood cells. The use of filter paper as a separate barrier for the retention of the red blood cells from the serum or plasma which flows from a matrix allows for a variety of filtration formats where a series of matrices treated with agglutinating agents are interspersed with pieces of filter material. Among the types of filters contemplated for such use are filters comprised of derivatized or underivatized cellulose, nylon, natural or synthetic membranes, or porous polyethylene matrices characterized by a pore size such that individual or agglutinated red blood cells will be retained by the porous matrix. Where more than one matrix is used, pore diameters are chosen to promote flow from one region to another.

Figures 3-5 are depictions of exemplary devices used to separate plasma from whole blood according to the second embodiment of the present invention. As illustrated in Figure 3, an apparatus (50) comprises a housing (52) which has an entry port (53) and an exit vent (56). Located within the housing (52) is a device (57) comprising a porous polyethylene matrix (58) and a paper matrix (66). The matrix (58) contains an agglutinating agent, has an inlet (54) and an outlet (55), and is in liquid receiving relationship with the paper matrix (66). The paper matrix contains a final red blood cell filtration region (60), an analyte reagent region (62), and a quantitative analysis region (64).

The device of the present invention may also comprise a novel means of collecting and retaining a predetermined amount of plasma or serum for analysis in a diagnostic assay. The novel means involves a metering matrix which enables the reproducible collection of discrete amounts of plasma or serum. This process is enabled by the use of a sintered porous matrix material which is selected for the following characteristics: a reproducible fluid uptake capacity that is proportional to the fixed dimensions of the matrix, a minimal reactivity with plasma or serum components, and a hydrophilic internal surface. These characteristics enable the matrix to collect and retain a predetermined volume of sample suitable for analysis in a diagnostic assay. Preferably, the matrix material is rigid for ease of handling, and optionally, the material is chosen as having the largest void capacity for the designated matrix dimensions. With such a matrix, the collection of the sample is independent of the level of the user's training, and there is no need for sophisticated measuring equipment.

A device of the present invention comprising such a collection or metering matrix can be used as components of diagnostic devices, such as flow-through and test strip devices, to collect a predetermined amount of sample that is not dependent upon the absorptive capacity of the paper, fiber and nitrocellulose materials typically used in such devices or upon the combined absorptive capacity of the device components. For example, in a test strip device the length of the strip typically determined the volume of sample which can be absorbed, and the dimensions of the test strip determine the amount of sample which will pass through the reaction and detection zones on the test strip. The metering matrix however, enables the collection and retention of a predetermined sample volume as well as the analysis of the entire sample volume, either within the matrix itself or within a sample receiver means to which the sample is transported, after the collection by the metering matrix of the total sample volume to be analyzed.

There are several different materials which can provide a volume measuring characteristic. These materials include paper, derivatized cellulose, porous plastic membranes and sintered porous materials. However, not all of these materials are equally suitable for use as metering matrices in diagnostic devices. For example, while a paper matrix may have the capacity to collect a sample of sufficient volume, paper matrices showed poor reproducibility in collecting that sample volume. Nylon matrices also have unacceptable reproducibility. The poor producibility of such matrices was attributed to the less sturdy and less resilient nature of such materials in withstanding handling stresses. Conversely, while matrices made from Nitrocellulose (Micron Separated, Inc., Westburough, Massachusetts) and Ultrabind (Gelman Sciences, Ann Arbor, Michigan) exhibit suitable reproducibility in volume metering, these materials are not suitable for the manufacturing of matrices of sufficient thickness to collect and retain a quantity of sample typically needed for analysis. Porous sintered materials, however, possess the structural rigidity and the void capacity to meet these needs.

Other features of the selected material which are important to collection or metering matrix performance include the particle size and pore size of the sintered material used to form the matrix. For example, a suitable metering matrix pore size was found to relate to the configuration of the device in which the matrix might be used. In a configuration where the metering matrix is situated directly below and in contact with the blood separator means, the flow dynamics through the matrix are less of a concern. If the matrix is situated laterally to the blood separator means, and a transfer material such as a wicking layer or strip is used to transport the plasma from the separator means to the matrix, then the pore size of the matrix should be large enough to induce sample collection by the matrix while maintaining even sample distribution within the matrix. The pore size of the matrix, however, cannot be too large in comparison to that of the strip. A large difference in capillary diameter becomes a dominant factor in flow resistance; the sample may wick along the fine pore channels but courser channels could be bypassed. When the wicking strip material is cellulose or a cellulose derivative, a metering matrix pore size in the range of about 5 μm to about 100 μm is typically used. Preferably, a pore size in the range of about 10 μm to about 25 μm is used. A most preferred pore size is about 15 μm, estimated by a mercury intrusion method, which is graded as "fine" pore. Pore sizes in the range of about 5 μm to about 10 μm are usable as super fine pore sizes in metering matrix materials. In addition, if such a lateral configuration is used with a wicking strip to transport the sample from the blood separator means to the collection or metering matrix, the filling of the matrix can be maximized by directing the sample flow to the matrix. The term "directing the flow" refers to the placement of the matrix at the end of the wicking strip or over a slit or space in the strip such that the entire adjacent surface of the matrix does not directly contact the strip material, i.e., a substantial portion of the matrix surface is not in physical contact with the strip. By using this directed flow format, the sample is deterred from bypassing the matrix and continuing through the strip material.

In an alternative embodiment, the metering matrix material can be modified to alter its manufactured pore size. For example, a porous matrix of sintered polyethylene which has been manufactured to have a certain nominal pore size can be coated with a treatment material, such as dextran, polyethylene glycol or carboxylatex, to produce a matrix which has sample collection and flow attributes characteristic of finer pored matrices. By treating the matrix, the void capacity of the matrix can be decreased and the flow rate through the matrix can be changed to simulate the characteristics of a matrix having a smaller pore size.

Another desired attribute of the collection matrices is the hydrophilic nature of the matrices. However, because sintered materials are generally not hydrophilic, matrices made of sintered material are rendered hydrophilic by treatment with surfactants, as described above in the treatment of the blood separator means. Surfactant solution concentrations of about 0.1% to about 0.5% are used to treat matrices of sintered polyethylene and thereby improve the performance of the matrices. With excess surfactant, the addition of sample can dissolve the surfactant and generate foam which could block the pores and capillaries of the matrix. With not enough surfactant or an uneven distribution of surfactant, there can be hydrophobic pockets within the matrix through which plasma does not readily flow.

In another embodiment, the plasma or serum sample collected and retained by the matrix can be eluted from the matrix by the addition of a buffer. The eluted sample and buffer can be gathered by any suitable receiving means. For example, the sample can be eluted into a test tube, cuvette or reaction well or onto a slide. Optionally the receiving means can contain all or some of the reagents necessary to perform the diagnostic assay of the sample. Alternatively, the matrix can be contacted to a receiving means such as an absorbent material will have a pore size smaller than that of the matrix, to induce the transport of sample from the matrix. Any suitable absorbent material can be used, such as a chromatographic, bibulous, porous or capillary material or other conventional absorbent material well-known to those skilled-in-the-art, and the material can optionally contain all or some of the reagents necessary to perform the diagnostic assay. The sample receiver means of a diagnostic device can be in direct contact with the collection matrix throughout use or it may be brought into contact with the matrix after the matrix has collected the predetermined volume of test sample.

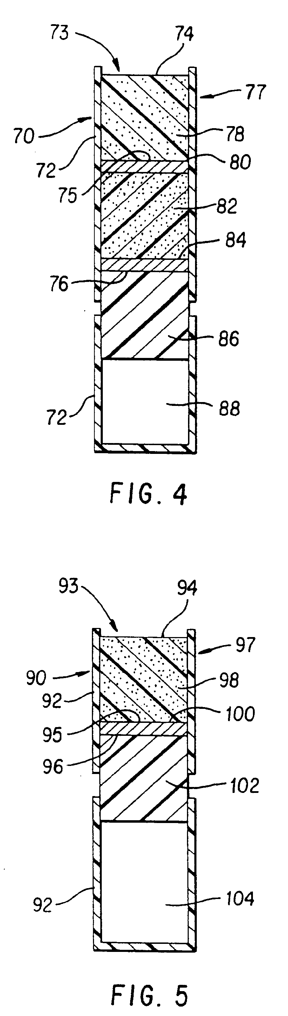

Generally, the metering matrix is enclosed or housed within a nonabsorptive casing material such that a matrix inlet port and, optionally, an exit port are defined. Such devices are illustrated in Figures 4 and 5. Figures 4 and 5 also illustrate the use of the collection matrix in conjunction with the blood separator means. In a vertical device configuration as shown in Figures 4 and 5, the housing material is chosen to minimize the effects of gravity upon the transport of sample from the blood separator means to the collection or metering matrix. The effects of gravity were minimized in the present invention by molding the housing from a material which has minimal interaction with plasma or serum. Suitable housing materials include polystyrene, acrylic, polycarbonate, teflon, polypropylene, polyethylene and silicon. A particularly preferred housing material is KR003 resin, a styrene-butadiene copolymer (Phillips 66, Bartlefville, Texas), due to its minimal interaction with plasma. Such a housing also prevents the overfill of the collection matrix by minimizing the plasma contact between the collection matrix and the housing. With the use of a housing, a matrix of given dimensions and nominal pore size will provide a reproducible void capacity and reproducible flow results, as demonstrated in the examples which follow.

As illustrated in Figure 4, an apparatus (70) comprises a housing (72) which has an entry port (73) and an exit port (76). Located within the housing (72) is a device (77) comprising a first porous polyethylene matrix (78), a first filter means (80), a second porous polyethylene matrix (82), and a second filter means (84). The first matrix (78) contains an agglutinating agent and has an inlet (74) and an outlet (75). Because the first filter means (80) is sandwiched between the first matrix (78) and the second matrix (82), the top of the first filter means (80) is in liquid receiving relationship with the outlet (75) of the first matrix (78), and the bottom of the first filter means (80) is in liquid receiving relationship with the top of the second matrix (82). The bottom of the second porous polyethylene matrix (82) is then in liquid receiving relationship with the top of the second filter means (84). Prior to addition of a blood sample to the device, it is placed on top of and in liquid receiving relationship with a third porous polyethylene matrix (86). This third matrix (86) is designed to retain and receive within its void space a selected predetermined volume of plasma which is then washed into a receiving cuvette (88). The third matrix (86) may contain some of the reagents necessary for the determination of a specific blood analyte while the cuvette (88) may contain other components of the reagent system.

As illustrated in Figure 5, the device (90) comprises a housing (92) which has an entry port (93) and an exit port (96). Located within the housing (92) is a device (97) comprising a porous polyethylene matrix (98) and a filter means (100). The matrix (98) contains an agglutinating agent and has an inlet (94) and an outlet (95). The top of the filter means (100) is in liquid receiving relationship with the outlet (95) of the matrix (98). Prior to addition of a blood sample to the device (90), it is placed on top of and in liquid receiving relationship with a second porous polyethylene matrix (102). This second matrix (102) is designed to receive and retain a selected predetermined volume of plasma which is then washed into a receiving cuvette (104). The second matrix (102) may contain some of the reagents necessary for the determination of a specific blood analyte while the cuvette (104) may contain other components of the reagent system.

The plasma or serum which flows from the devices of the present invention may flow directly into a receiving matrix. Among the different types of matrices available which may receive the plasma or serum from the device are a dye paper matrix to which the analytical reagents have been attached or porous matrices made from sintered materials, such as glass, steel, ceramics, or plastic polymers, which are capable of retaining a selected volume of plasma or serum. According to use of the dye paper matrix, the plasma or serum enters the paper and flows as a front through the paper. It comes in contact with the analytical reagents incorporated in the paper and the assay for the desired blood component is performed on the paper.

The preferred sintered matrix capable of receiving the flow of plasma or serum from the device is a treated porous polyethylene matrix. The plasma or serum flows from the device and a selected amount enters the receiving matrix. The void space of the receiving matrix determines the volume of plasma or serum which may enter the receiving matrix. The plasma or serum is eluted from the receiving matrix into a cuvette by addition of an elution buffer. Analysis of the desired blood component occurs within the cuvette which may contain the desired analytical reagents. The porous polyethylene matrix may also contain reagents necessary for the analysis of the analyte after plasma or serum have been eluted. Such an analysis may take place in the polyethylene matrix or the sample and reagents may be eluted into the cuvette for subsequent reading.

Although the devices of the present invention may be used generally as a means for providing plasma or serum for use in other diagnostic procedures, various analytical reagents may be incorporated into the devices in order to render them suitable for carrying out an analysis for a selected component of blood plasma or serum. Among those contemplated are the reagents such as those utilized for carrying out enzymatic analysis of analytes such as cholesterol, triglycerides, and glucose in the blood. It is contemplated that reagents for a wide variety of assays may be incorporated into the devices of the present invention. Examples of reagents which can be incorporated into a matrix according to another embodiment of the present invention, include buffers, acids, bases, enzymes, enzyme substrates, reagents useful for assay methodologies and reagents useful in chemical analyses. Also, biologically active molecules and components including antigens, antibodies, recombinant proteins, plasmids, fragments of each of these, and the like also can be contained in the matrix.

The porous matrices of the invention will retain serum or plasma in their interstices in proportion to the volume of the porous matrix. Red blood cell free plasma or serum will generally remain in the interstices of the porous matrix unless it is removed by external means. Such external means can include the use of positive hydrostatic pressure such as may be obtained by application of additional blood sample or elution buffer to the matrix. Alternatively, filter means such as filter paper or additional porous matrices in liquid receiving relationship with the matrix may be used to induce the flow of plasma or serum by capillary action out of the matrix. Accordingly, it is desired to use the smallest matrix consistent with flow and purity considerations in order to maximize serum or plasma yield.

The rate of flow of plasma and serum through the porous matrix may be controlled by varying the porosity and flow properties of the contacting filter means. It is contemplated that filter means may be selected to induce rapid flow through the porous matrix. Alternatively, where it is desired to maintain a longer residence time of blood sample within the porous matrix, a filter means providing a relatively slower rate of fluid flow out of the porous matrix may be selected. It is contemplated that slowing the rate of flow through the porous matrix can increase the efficiency of agglutination within the matrix. It is further contemplated that use of a filter means inducing a relatively slow rate of fluid flow can provide the advantage of greater agglutination efficiency and may also allow use of a smaller porous matrix thus providing the additional advantage of maximizing plasma or serum yield.

According to a preferred embodiment of the present invention, the pore size of the matrix is selected in conjunction with the length and volume of the matrix, void volume of the matrix, volume and concentration of the solution for the reagent delivery system. These factors determine the amount of recoverable dry reagent present in each matrix.

According to one method of making the reagent delivery system, a known volume of a component was mixed into solution of a reagent solution of a known concentration. Any final adjustments, such as pH adjustment and filtration, were performed. Next, a known quantity of matrices were added to the solution and mixed. The quantity of matrices which was saturated with component solution was determined as follows. First, the capacity of the matrix, the so-called void volume of the matrix, was determined by measuring the recoverable amounts of dry reagents, which methods are known to those of ordinary skill in the art. Then, calculations were performed to determine the quantity of matrices which were to be saturated with the component solution. The matrices were allowed to saturate. It should be noted that a vacuum may be drawn to ensure complete loading of the matrix with the reagent component. Next, the matrices were frozen by placing the container on dry ice. After freezing, the matrices were lyophilized according to methods known by those of ordinary skill in the art.

The device of the invention thus may comprise a reagent delivery system wherein a reagent is provided in a "unitized" form recoverable upon contact with a liquid. Reagents can be added to matrices under conditions most favorable to their recovery.

The following specific examples are directed to several embodiments of the present invention and are not to be construed as limiting the scope of the invention.

Example 1

The device depicted in Figure 2 contains a first matrix which has dimensions of 5 mm x 4 mm x 3 mm, is treated with a wetting agent and has adsorbed to it a 30 microliter solution of 5 mg/ml anti-red blood cell antibodies in 100 mM citrate, pH 5.6. The pore size of the first matrix and the agglutinating agents adsorbed to it are selected to retain substantially all red blood cells within the matrix. Loading is accomplished by saturating the first matrix with the antibody solution. Once the matrix is loaded, it is frozen and lyophilized. The second matrix, which has dimensions of 6 mm x 4 mm x 0.8 mm, is treated with a wetting agent and contains the reagents necessary for determination of an analyte in the plasma. Whole blood is added through the entry port and, as it percolates through the first matrix, red blood cells within the sample are agglutinated by the anti-red blood cell antibodies and the clumps are filtered out. The plasma, now free of red blood cells, flows from the first matrix into the second matrix and solubilizes the enzymes and dye component of the reagent system located there. This mixture then flows into the dye paper matrix, where determination of the analyte occurs by reaction of the blood analyte with other enzymes and dye components of the reagent system.

Example 2

The device depicted in Figure 3, which has dimensions of 6 mm x 4 mm x 0.8 mm, is treated with a wetting agent and has adsorbed to it 8 microliters of a 5 mg/ml solution of anti-red blood cell antibodies: IgG fraction (Organon Teknika Corp., Cappel Division), in 100 mM citrate buffer, pH 5.6. Loading is accomplished by applying the antibody solution to the matrix under vacuum. Once the matrix is loaded, it is frozen and lyophilized. Whole blood is added through the entry port and, as it percolates through the matrix, red blood cells within the sample are agglutinated by the anti-red blood cell antibodies and the red blood cells are partially filtered out. Final red blood cell filtration occurs in the filtration region of the dye paper matrix. As the plasma continues flowing up the dye paper matrix, it contacts the analyte reagent region where the reagents for analyte determination have been lyophilized. The plasma solubilizes these reagents and quantitation of the analyte by reaction of the sample and reagents occurs in the quantitative analysis region.

Example 3

In this example, the device disclosed in Example 2 was used to separate plasma from whole blood so that a blood cholesterol assay could be performed. The matrix was loaded with a solution of 8 microliters of 5 mg/ml anti-red blood cell antibodies: (IgG fraction (Organon Teknika Corp., Cappel Division)), 10 mg/ml cholesterol esterase, 10 mg/ml horseradish peroxidase, and 5 mg/ml 4-aminoantipyrine in 100 mM citrate, at pH 5.6. The device was placed on top of and in contact with a dye paper matrix and whole blood was added to the device through the entry port. As the blood percolated through the porous matrix, red blood cells within it were agglutinated by the anti-red blood cell antibodies and the red blood cells were partially filtered out by the matrix. Final red blood cell filtration occurred in the region of the dye paper matrix which was 5-6 mm from the paper origin where the device contacted the dye paper matrix. At this point 5-6 mm from the paper origin, the plasma contacted an analyte determinator region which was a 3 mm wide zone that contained a solution of 100 mg/ml cholesterol oxidase, 1% (w/v) triton X-100, and 100mM NaPO$_4$, at pH 6.8. As the plasma flowed up the paper matrix it solubilized the lyophilized reagents. The flow continued into the dye paper matrix where quantitation of the analyte (cholesterol) occurred.

Example 4

With respect to the device depicted in Figure 1, the matrix was treated with a wetting agent and had adsorbed to it various 25 microliter solutions of anti-red blood cell antibodies: IgG fraction (Organon Teknika Corporation, Cappel Division), in 20 mM citrate buffer, pH 5.6. In this device, the data listed in Table 1 below indicated that a 2 mg/ml antibody concentration loaded under vacuum was optimal for filtering out red blood cells from whole blood having a hematocrit of 30-60% red blood cells and releasing at least 5 microliters of plasma from a 25 microliter sample of whole blood. Hematocrit refers to the percentage of the volume of a blood sample occupied by red blood cells. For example, a 25 microliter blood sample with a hematocrit of 30 contains 7.5 microliters of red blood cells and 17.5 microliters of plasma. The whole blood samples were treated with heparin as an anticoagulant. This concentration of antibody allowed plasma to flow 12 mm to the end of the filter paper in a reasonable amount of time, while still retaining substantially all the red blood cells of a sample within the matrix. Higher antibody concentrations resulted in greater agglutination which blocked the pores within the matrix and precluded flow of plasma. The pore size of the matrix and the agglutinating agents adsorbed to it were selected to retain substantially all red blood cells within the matrix. Loading was accomplished by applying the antibody solution to the matrix by saturation, i.e. soaking the matrix in solution, or under vacuum, i.e. soaking the matrix in solution and pulling a vacuum on it for 10 minutes. It was determined that loading under vacuum was superior to loading by saturation because vacuum loading ensures that no air pockets remain in the matrix after loading. Loading by saturation does not ensure this same result. Once the matrix was loaded, it was frozen and lyophilized. The trials were run in sets of 6 and a trial was determined to be "substantially free" of red blood cells (i.e. "No RBCs") by the visual determination that there were no red blood cells in the filter paper, which was in liquid receiving relationship with the matrix, after the filtration step. In the table, "seconds to end" refers to the elapsed time from the addition of a blood sample to the inlet of the matrix, until plasma reached the end of the filter means.

## TABLE 1

ANTI-RED BLOOD CELL ANTIBODIES (mg/ml)

| | 1 | 2 | 3 | 4 | 5 |
|---|---|---|---|---|---|
| **HEMATOCRIT = 30** | | | | | |
| *Saturated* | | | | | |
| seconds to end | 74.5 | 82.0 | 104.2 | 159.7 | 219.3 |
| no RBCs | 2/6 | 5/6 | 6/6 | 6/6 | 5/5 |
| *Vacuum* | | | | | |
| seconds to end | 65.3 | 70.2 | 105.2 | 84.2 | 108.8 |
| no RBCs | 2/6 | 4/6 | 6/6 | 6/6 | 6/6 |
| | | | | | |
| **HEMATOCRIT = 45** | | | | | |
| *Saturated* | | | | | |
| seconds to end | 200.0 | 290.0 | 585.6 | 631.7 | 469.8 |
| no RBCs | 5/6 | 6/6 | 5/5 | 5/5 | 5/5 |
| | | | | | |
| **HEMATOCRIT = 45** | | | | | |
| *Vacuum* | | | | | |
| seconds to end | 107.0 | 326.7 | 193.0 | 414.8 | n.f. |
| no RBCs | 6/6 | 4/4 | 5/5 | 6/6 | n.f. |
| | | | | | |
| **HEMATOCRIT = 60** | | | | | |
| *Saturated* | | | | | |
| seconds to end | 336.7 | 700.7 | n.f. | n.f. | n.f. |
| no RBCs | 2/3 | 3/3 | n.f. | n.f. | n.f. |
| | | | | | |
| *Vacuum* | | | | | |
| seconds to end | 354.3 | 824.5 | n.f. | n.f. | n.f. |
| no RBCs | 5/5 | 4/4 | n.f. | n.f. | n.f. |

n.f. = no flow of plasma to end of filter paper matrix.

At a low blood hematocrit (e.g. 30), up to 15 microliters of plasma may be released from a 25 microliter sample of whole blood in as little as 2 minutes, while at a high blood hematocrit (e.g. 60), about 5 microliters of plasma may be released from a 25 microliter sample of whole blood in around 15 minutes.

Example 5

In this example, the device disclosed in Example 3 was used to separate plasma from whole blood so that an assay to detect antibodies to Human Immunodeficiency Virus (HIV) could be performed. The matrix was loaded with 8 microliters of 5 mg/ml anti-red blood cell antibodies: (IgG fraction (Organon Teknika Corp. Cappel Division)), 5 microliters detector label prepared by binding 10 micrograms/ml HIV antigen with 0.05% black latex poly(pyrrole), in aqueous suspension at pH 7.0. The device was placed on top of and in contact with a 3 x 30 mm strip of nitrocellulose (S & S, Keene, NH), which had a 5 micrometer pore size, and 30 microliters of whole blood were added to the device through the entry port. As the blood percolated through the porous matrix, red blood cells within it were agglutinated by the anti-red blood cell antibodies and the red blood cells were partially filtered out by the matrix. The plasma in the sample mixed with the label suspension in the matrix and then entered the nitrocellulose strip where final red blood cell filtration and analysis of the separated plasma occurred.

Example 6

To the device depicted in Figure 4, whole blood is added through the entry port. As the blood percolates through the first matrix, red blood cells within the sample are agglutinated by the anti-red blood cell antibodies and the red blood cells are partially filtered out. The remaining red blood cells and smaller clumps of agglutinated red blood cells pass into the first filter where additional separation of plasma from the red blood cells occurs. Those red blood cells not retained by the first filter pass into the second matrix where additional separation of plasma from the red blood cells occurs. Finally, any red blood cells not retained by the second matrix pass into the second filter, to which at least one red blood cell agglutinating agent has been adsorbed, where agglutination of the remaining red blood cells in the plasma occurs. The plasma then flows into the receiving matrix where the plasma volume is quantitated. The red blood cell filtration stack is separated from the receiving matrix and the selected volume of plasma is eluted into an attached cuvette by the addition of the elution buffer. The cuvette may contain various analytical reagents. Thorough mixing of the plasma and the elution buffer is accomplished by inverting the cuvette 2 times. After a specified waiting period, the results of the test are obtained by comparing the color of the liquid with a standard chart.

Specifically, with respect to the device depicted in Figure 4, the pore size of the first matrix (Porex 4897) and the agglutinating agents adsorbed to it were selected to agglutinate and retain most, but not all, red blood cells within the first matrix. The first matrix is molded into a cylindrical shape having the dimensions of 0.5 cm (0.2 inches) in diameter and 0.18 cm (0.07 inches) in length and had adsorbed to it 15.0 microliters of a solution of 0.44% (w/v) antiserum to red blood cells (Organon Teknika Corp., Cappel Division), 4.4% (w/v) Polybrene (Aldrich Fine Chemicals), and 4.4% (w/v) PVP (Aldrich Fine Chemicals) in 0.35 mM citrate buffer, pH 7.4 (Fisher Chemicals). The coated first matrix was dried in a hot air oven. The composition of the solution and the quantity loaded into the first matrix were chosen to provide very rapid red blood cell agglutination without causing red blood cells to lyse and without causing hemodilution. The remaining red blood cells were removed from the plasma by passing it through the first filter means (Whatman 31 ET), the second porous polyethylene matrix (Porex® 4932), and the second filter means (Whatman 31 ET). This last filter means had incorporated within it 36.1 microliters/cm² of a 1 mg/ml solution of antiserum to red blood cells. The coated last filter means was dried in a hot air oven. This device produced 15 microliters of clear plasma 99% free of hemoglobin from 50 microliters of blood within 3 minutes as shown in Table 2. Upon removal of the red blood cell filtration stack, i.e. the first matrix, the first filter means, the second matrix, and the second filter means, the plasma was eluted into a cuvette by the addition of an elution buffer.

Example 7

To the device depicted in Figure 5, whole blood is added through the entry port. As the blood percolates through the matrix, red blood cells within the sample are agglutinated by the anti-red blood cell antibodies and the red blood cells are partially filtered out. The remaining red blood cells and smaller clumps of agglutinated red blood cells pass into the filter where additional separation of plasma from the red blood cells occurs. The plasma then flows into the receiving matrix where the plasma volume is quantitated. The red blood cell filtration stack is separated from the receiving matrix and the selected volume of plasma is eluted into an attached cuvette by the addition of the elution buffer. The cuvette may contain various analytical reagents Thorough mixing of the plasma and the elution buffer is accomplished by inverting the cuvette 2 times. After a specified waiting period, the results of the test are obtained by comparing the color of the liquid with a standard chart.

Specifically, with respect to the device depicted in Figure 5, the pore size of the matrix (Porex 4897) and the agglutinating agents adsorbed to it were selected to agglutinate and retain most, but not all, red blood cells within the matrix. The matrix had adsorbed to it 15.0 microliters of a solution of 0.88% (w/v) antiserum to red blood cells (Organon Teknika Corp., Cappel Division), 1.76% (w/v) Polybrene (Aldrich Fine Chemicals), and 1.76% (w/v) PVP (Aldrich Fine Chemicals) in 0.397 mM citrate buffer, pH 7.4 (Fisher Chemicals). The coated matrix was dried in a hot air oven. The composition of the solution and the quantity loaded into the matrix were chosen to provide very rapid red blood cell agglutination without causing red blood cells to lyse and without causing hemodilution. The remaining red blood cells were removed from the plasma by passing it through the filter means (Whatman 1CHR). The filter means had incorporated within it 15.0 microliters/cm² of a 1 mg/ml solution of antiserum to red blood cells. The coated filter means was dried in a hot air oven. This device produced 10 microliters of clear plasma 99% free of hemoglobin from 40 microliters of blood within 2 minutes as shown in Table 3. Upon removal of the red blood cell filtration stack, i.e. the matrix and the filter means, the plasma was eluted into a cuvette by the addition of an elution buffer.

TABLE 2

BLOOD SEPARATOR PERFORMANCE OF FOUR LAYER STACK
(POREX-31ET-POREX-31ET. POREX)

| Receiving Frit Dimension | | Blood Sample Volume | Hematocrit of Sample | Time | | | Plasma Volume Quantitate |
|---|---|---|---|---|---|---|---|
| Diameter | Thickness | | | Plasma Appeared in Frit | Frit Appeared Full | Device Separated | |
| (mm) | (mm) | (µL) | (%) | (Seconds) | (Seconds) | (Seconds) | (µL) |
| 3.5 | 3.4 | 50 | 25 | 10 | 60 | 120 | 15.5 |
| | | 50 | 38 | 15 | 90 | 120 | 15.3 |
| | | 50 | 50 | 20 | 120 | 180 | 15.1 |
| | | 50 | 75 | 60 | NO | 900 | about 5 |
| | | 75 | 75 | 50 | NO | 600 | about 10 |

TABLE 3

BLOOD SEPARATOR PERFORMANCE OF TWO LAYER STACK
(POREX-1chr.POREX)

| Receiving Frit Dimension | | Blood Sample Volume | Hematocrit of Sample | Time | | | Plasma Volume Quantitate |
|---|---|---|---|---|---|---|---|
| Diameter (mm) | Thickness (mm) | (µL) | (%) | Plasma Appeared in Frit (Seconds) | Frit Appeared Full (Seconds) | Device Separated (Seconds) | (µL) |
| 3.5 | 1.7 | 35 | 35 | 10 | 30 | 120 | 8.7 |
| | | 35 | 40 | 10 | 30 | 120 | 8.6 |
| | | 35 | 45 | 10 | 40 | 120 | 8.5 |
| | | 35 | 50 | 15 | 40 | 120 | 8.4 |
| | | 40 | 55 | 15 | 50 | 120 | 8.3 |
| | | 50 | 60 | 20 | 60 | 120 | 8.2 |

Example 8

Several methods can be used to estimate the void volume and the metering capacity of the collection matrices. When the materials are thin and have different appearances when dry or wet, a diffusion wicking method was used. In this method, a fixed volume of sample was applied to the surface of the matrix by precision pipetting. The diameter of the wet spot on the matrix surface, following the diffusion process, provided information about the volume capacity and reproducible sample collection of the matrix material.

Chromatography paper matrices (31ET cellulose paper, Whatman Applied Technology Business, Kent, England) had about the same volume capacity as nitrocellulose matrices (5.0 µm pore, Micron Separated, Inc.), but the reproducibility of sample collection was better with the paper matrices (2% variation) as compared to the nitrocellulose (3.4% variation). Ultrabind membrane matrices (Gelman) demonstrated good reproducibility (2% variation), but these matrices had a low volume capacity due to the thinness of the material. While it is

15

possible to coat nitrocellulose to form a thicker matrix material, such matrices were extremely fragile.

When the material of interest was not translucent, and when a determination of the total saturation capacity of the material was desired, a method of weighing the matrix before and after sample collection was used. The matrix was cut to a uniform dimension (discs having a surface area of 1.13 square centimeters) and weighed, and the matrix was soaked in the sample. The matrix was then removed from the sample, and the excess sample was removed from the matrix surface by quick-blotting with a coarse-pore material such as cellulose (Kim Wipe, Kimberly Clark, GA). The difference in weight between the wetted and the dry matrix material indicated the void capacity of the matrix. Table 4 illustrates the results of the void capacity determinations for several different matrix materials, including sintered polyethylene (Porex 4897, Porex Technologies, Inc, Fairburn, GA) and nylon (nylon mesh, Spectrum Medical Industries, Inc., Los Angeles, CA).

<u>Table 4</u>

<u>Void Capacity by Wet-Blot-Weighing Method</u>

| Material | Thickness (mm) | Dry Weight (mg) | Void Capacity (mg) | Void Variation (%) |
|---|---|---|---|---|
| Nitrocellulose | 0.17 | 3.8 | 17.1 | 3.5 |
| Nylon | 0.05 | 4.0 | 3.2 | 29.0 |
| Porex 4897 | 1.4 | 72.9 | 64.3 | 1.0 |
| Ultrabind | 0.17 | 7.5 | 5.9 | 3.5 |
| Whatman Paper | 0.4 | 20.5 | 40.5 | 3.9 |

A comparison of the results presented in Table 4 illustrates that the sintered polyethylene matrix material had the largest void capacity for matrix size as well as the least variation in measurements. The nylon matrix had unacceptable reproducibility. The nitrocellulose and Ultrabind matrices had an insufficient capacity for large sample volumes. While multiple layers of the latter materials could be stacked to bring the void capacity into the desired range, the reproducibility of volume metering with such stacks was less well controlled.

Example 9

This experiment demonstrated a method for the modification of the pore size of a matrix. The modification was performed by treating the matrices with a solution of dextran (2.5% in water) . The effect of coating the internal surface area of the matrix with dextran was determined by applying plasma samples having different hematocrit values to the matrices. The results of the experiment are presented in Table 5. The data show the effects of pore size and dextran coating on plasma recovery using vertical configuration devices, as shown in Figures 4 and 5, using similar housings. The entries are expressed as a percent of the plasma obtained from a 45% hematocrit blood specimen.

## Table 5

### Effects of Pore Size and Matrix Coating

| Blood sample Hematocrit (%) | Uncoated Large Pore Receiver (25 μm) | Coated Large Pore Receiver (25 μm) | Uncoated Small Pore Receiver (10 μm) |
|---|---|---|---|
| 0 | 150 | 132 | 110 |
| 30 | 112 | 118 | 105 |
| 45 | 100 | 100 | 100 |
| 50 | NT | 95 | 98 |
| 55 | 87 | 89.1 | 95 |
| 60 | NT | 80.4 | 90 |
| Volume (μL) at 45% hematocrit | 9 | 6.8 | 4.5 |

NT – not tested

### Example 10

Experiments were performed using different materials to manufacture the housing for the collection matrix. The most suitable housing materials were those that minimized the interaction of the housing with the plasma or serum sample. The collection matrix had the tendency to over-fill when the blood samples had a low hematocrit, because there was minimal flow resistance in the material. The collection matrix had a tendency to under-fill when the blood samples had a high hematocrit, due to the increased amount of materials in the sample which could block the pores of the collection matrix. Table 6 presents data comparing the plasma volume received and the percent recovered from collection matrices in housings made of different materials. Plasma specimens were used as test samples, and the void capacity of the sintered polyethylene collection matrix was 8.5 μL, as estimated by the wet-blot-weigh method. The expected 8.5 μL was obtained by measuring the plasma weight using blood specimens of 45% hematocrit. The housings were made from a styrene-butadiene copolymer (KR003, Phillips 66), a styrene-acrylic alloy (Q886, Monsanto, MO), an acrylo-butyl styrene (ABS, Monsanto, MO), polymethylpentene (TPX, Mitsui Petrochemical, New York, NY), polypropylene (PD-213, Himont, Bloomington, DE) or polyethylene (PE, GE Plastics, Pittsfield, MA).

EP 0 392 377 B1

### Table 6

### Housings to Minimize Matrix Over-fill

| Plastic Resin | Plasma Volume Received (uL) | % Recovery over the expected 8.5 uL |
|---|---|---|
| KR003 | 9.8 | 115 |
| Q886 | 10.6 | 124 |
| ABS | 11.0 | 129 |
| TPX | 11.6 | 136 |
| PD-213 | 10.9 | 129 |
| PE | 11.6 | 136 |

Table 7 presents plasma recovery data using collection matrices in housings made of different materials and whole blood samples of 32% hematocrit. The percent recovery was calculated from the plasma recovered from whole blood samples of 32% hematocrit in comparison to that recovered using 43% hematocrit blood as a specimen, assuming 43% hematocrit to be "100% volume".

### Table 7

### Effect of Housing on Plasma Recovery

| Housing Material | % Plasma Recovered |
|---|---|
| KR003 | 109 |
| TPX | 111 |
| PD-213 | 120 |
| PE | 114 |

The housings made of KR003 resin demonstrated the least plasma recovery variations with samples of varied hematocrit.

It is also possible to minimize the over-filling effect and hematocrit effect on plasma recovery by coating the molded plastic housings with detergent to form a hydrophobic surface or by a siliconization process using curable silicon (MDX4-4159, Dow-Corning, Midland, Michigan).

Example 11

This experiment compared the effects of directed flow and undirected flow in diagnostic device configurations. The blood separation means was a sintered polyethylene cylinder having the dimensions of 0.188 cm (0.074 inch) in length and 0.493 cm (0.194 inch) in diameter. The pore size of the blood separator, and the agglutinating agents absorbed within the separator, were selected to rapidly agglutinate and retain within the matrix most of the red blood cells from the whole blood sample without causing hemodilution and without causing the cells to lyse. The separator had been saturated with a solution of 8.89 optical density (280 nm)/mL antiserum to red blood cells (Organon Teknica Corporation, Durham, NC) in citrate buffer (0.397 mM, pH 7.4, Fisher Chemicals, Fairhaven, PA) and surfactant (0.1% Triton X-405, Sigma, St. Louis, MO) The separator means was then dried in a hot air oven.

The metering or plasma collection matrix was also a sintered polyethylene cylinder, having the dimensions of 0.178 cm (0.070 inch) in length and 0.381 cm (0.150 inch) in diameter. The cylinders were made by means of a hollow core punch to remove matrices of uniform size from a stock sheet of sintered polyethylene having a five micron nominal pore size (General Polymeric). The plasma collection matrix had been made hydrophilic by pretreating the sheet with a three percent suspension of carboxylatex (Seradyne, Indianapolis, IN) in me-

18

thanol, followed by overnight drying under vacuum.

The wicking strip was composed of cellulose paper (Schleicher & Schuell #410, Keene, NH)

A comparison of devices was performed using an undirected flow device configuration in which the entire bottom surface of the metering matrix was in contact with the upper surface of the wicking layer, and a directed flow device configuration in which a substantial portion of the bottom surface of the metering matrix was positioned over a slit or space in the upper surface of the wicking layer or strip. Blood samples were applied to the blood separator means of the devices, wherein the red blood cells were removed and the resultant plasma entered the wicking layer by capillary force. The plasma continued through the wicking strip to the collection matrix and beyond to an overflow portion of the wicking strip. When no further movement of plasma through the overflow zone was visually observed, the collection matrix was removed and weighed. By comparing the weights of the used matrices to the known starting weights of the matrices, a net increase in weight due to plasma collection was calculated. The results are present in Table 8. The results indicate that when the lateral flow device configuration was used, the directed flow configuration provided a more uniform filling of the collection matrix over a broad hematocrit range. Under-filling of the matrix was observed with the undirected flow configuration at a 55% hematocrit.

<u>Table 8</u>

<u>Net Increase in Weight of Matrix Due to Plasma Collection</u>

| | Blood Hematocrit (%) | | |
|---|---|---|---|
| <u>Configuration</u> | <u>0</u> | <u>30</u> | <u>55</u> |
| Undirected | 4.5 mg | 5.0 mg | 1.9 mg |
| Directed | 5.0 mg | 4.7 mg | 4.0 mg |

Example 12

The following experiment demonstrated the precision of the collection matrix and blood separator, the correlation of assay results with a reference assay and the effect of hematocrit on the performance of the assay device. A vertical blood separation stack, as illustrated in Figures 4 and 5 was used.

The blood separator material was prepared from sintered polyethylene (Porex 4897). The material was saturated with a solution of antiserum to human red blood cells (8.89 optical density [280 nm]/mL; Cappel 0101-1322, Organon Teknica Corporation) in citrate buffer (0.573 mM, pH 7.4, Fisher Chemicals) and surfactant (0.1% Triton X-405). The material was then dried at low, laminar air flow at room temperature.

The collection matrix material was sintered polyethylene sheet stock (General Polymerics) having a pore size of about 5 μm. The sheet was saturated with a 3% suspension of carboxylatex (Seradyne) in methanol. The solvent was then removed by holding the sheets under vacuum overnight in a desiccator.

Two discs, each 0.508 cm (0.200 inch) in diameter and 0.163 cm (0.064 inch) in length, were punched from the blood separator material and inserted into a cylindrical housing as shown in Figures 4 and 5. A disc of chromatography paper 0.508 cm (0.200 inch) in diameter (Whatman 1CHR) that had been coated in IgG to human red blood cells (Organon Teknica, Cappel 0201-1322) in sodium citrate buffer (2.0 mM, pH 7.4) was then placed in the housing. A disc of collection matrix material (0.138 inch in diameter, 0.163 cm (0.064 inch) in length) was punched from the matrix material. The disc dimension was chosen to provide a collection matrix with a void capacity of about five microliters. The collection matrix was inserted in the housing such that the bottom surface of the paper disc was in contact with the upper surface of the collection matrix, as illustrated in Figure 4.

The assay reagents were prepared to for the performance of a cholesterol assay. The reagents necessary to measure plasma cholesterol were delivered in a unitized format referred to as a "unit dose reagent". The unit dose reagent is a delivery format in which the assay reagents are formed into a soluble mass. When contacted with the appropriate buffer, the unit dose reagent dissolves to release the component reagents without leaving behind insoluble materials which can interfere with the spectrophotometric determination of color.

The cholesterol assay unit dose reagent contained: cholesterol ester hydrolase (6660 units, Amano, Troy, VA); cholesterol oxidase (940 units, Boehringer Mannheim Biochemicals, Indianapolis, IN); peroxidase (136,000 units, Amano); 4-aminoantipyrene (677 mg) and 3,5-dichlorohydroxybenzene sulfonate (2922 mg). The component reagents would react with the plasma sample to produce a red-colored reaction product, which can be read in a spectrophotometer (515 nm).

A reagent unit dose reagent was placed in a cuvette which was used as the sample receiver means of the diagnostic device, as described above and as depicted in Figures 4 and 5.

The assay was performed by placing a sample of whole blood (50 µL) on the top of the blood separator means. After about two minutes, the cell plasma separation was complete, leaving the collection matrix filled with plasma. The blood separator means was removed from the device, and plasma was eluted from the collection matrix into the cuvette by the addition of elution buffer (0.5 mL). The unit dose reagent dissolved upon the addition of plasma sample and buffer to the cuvette, thereby liberating the reagents necessary for the cholesterol assay. The absorbance of the resulting reaction mixture was read in a spectrophotometer.

The test was performed in duplicate using whole blood samples from 68 patients. The results of the assay were compared to assay results obtained with the Vision-Cholesterol Assay (Abbott Laboratories, Abbott Park, IL) . The overall precision of the system, i.e., the blood separator, metering matrix and unit dose reagent, was calculated by averaging the coefficient of variation (%CV) of the individual determinations. The overall precision of the assay was determined to be 3.3 %CV. The overall slope, intercept and correlation of each patient's cholesterol level determined by the present invention in comparison with the Vision assay determination were as follows: slope 0.93, intercept 14 and correlation 0.96. Therefore, the test device of the present invention provided acceptable precision and accuracy of assay results when correlated with the results of the reference method.

## EXAMPLE 13

### PREPARATION OF MATRICES CONTAINING GLUCOSE OXIDASE

A component solution was prepared which contained 250 mg/ml of glucose oxidase (available from Sigma Chemical Corp, St. Louis, MO) and 19 mg/ml of 4-aminoantipyrine (available from Sigma Chemical Co. St. Louis, MO). A quantity of Porex® LC06 WW5.3 matrices of sintered polyethylene, having a nominal matrix volume of approximately 6.5 µL (microliters) (available from Porex, Inc., Fairburn, Georgia), were saturated with this component solution, frozen and lyophilized according to the method of the invention.

## EXAMPLE 14

### PREPARATION OF MATRICES CONTAINING PEROXIDASE REAGENT

A component solution was prepared which contained 7.5 mg/mL of peroxidase (available from Sigma Chemical Co., St. Louis, MO) and 100 mg/mL of 3,5-Dichloro-2-benzene sulfonic acid (DCHBS) (available from Sigma Chemical Co., St. Louis, MO). A quantity of Porex® LC06 WW5.3 matrices of sintered polyethylene, having a nominal matrix volume of approximately 6.5 uL (available from Porex, Inc., Fairburn, Georgia) were saturated with this component solution, frozen and lyophilized according to the method of the invention.

## EXAMPLE 15

### ASSAY FOR GLUCOSE

Matrices prepared according to Example 1 and Example 2 were added to 3 mL of 20 mM Sodium Citrate (pH 5.6) in a container. The container was vortexed for approximately 3-5 seconds, after which time the contained reagents were delivered into solution. 5 µL of glucose standard (available from Sigma Chemical Co., St. Louis, MO), at approximately 100 mg/dL, nominal, was added to the mixture of buffer and matrices. The container was incubated for 30 minutes at 37° C. Absorbance was measured at 450, 512 and 560 nm by using distilled water as a blank. The standard was tested 8 times and a mean and standard deviation were calculated.

## EXAMPLE 16

### ASSAY FOR GLUCOSE

Matrices prepared according to Example 1 and Example 2 were added to 3 ml of 20 mM Sodium Citrate (pH 5.6) in a container. 5 uL of glucose standard (available from Sigma Chemical Co., St. Louis, MO) at approximately 300 mg/dL, nominal, was added to the mixture of buffer and matrices. The container was vortexed for approximately 3-5 seconds, after which time the contained reagents were delivered into solution. The container was incubated for 30 minutes at 37° C. Absorbance was measured at 450, 512 and 560 nm by using distilled water as a blank. The standard was tested 8 times and a mean and standard deviation were calculated.

EXAMPLE 17

ASSAY FOR GLUCOSE

Matrices prepared according to Example 1 and Example 2 were added to 3 ml of 20 mM Sodium Citrate (pH 5.6) in a container. The container was vortexed for approximately 3-5 seconds, after which time the contained reagents were delivered into solution. 5 uL of glucose standard (available from Sigma Chemical Co., St. Louis, MO) at approximately 800 mg/dL, nominal, was added to the mixture of buffer and matrices. The container was incubated for 30 minutes at 37 C. Absorbance was measured at 450, 512 and 560 nm by using distilled water as a blank. The standard was tested 8 times and a mean and standard deviation were calculated.

TABLE 9
ABSORBANCE OF GLUCOSE STANDARDS (mg/dL) AT 450nm

| SAMPLE | 0 | 100 | 300 | 800 |
|--------|--------|--------|--------|--------|
| 1 | 0.0807 | 0.1415 | 0.2228 | 0.4588 |
| 2 | 0.0895 | 0.1302 | 0.2265 | 0.4887 |
| 3 | 0.0818 | 0.1280 | 0.2094 | 0.4897 |
| 4 | 0.0818 | 0.1398 | 0.2217 | 0.4850 |
| 5 | 0.0818 | 0.1294 | 0.2104 | 0.5335 |
| 6 | 0.0867 | 0.1394 | 0.2457 | 0.4917 |
| 7 | 0.0820 | 0.1298 | 0.2155 | 0.4731 |
| 8 | 0.0864 | 0.1311 | 0.2074 | 0.4610 |
| MEAN | 0.0838 | 0.1337 | 0.2199 | 0.4852 |
| S.D. | 0.003 | 0.005 | 0.012 | 0.022 |

TABLE 10
ABSORBANCE OF GLUCOSE STANDARDS (mg/dL) AT 512 nm

| SAMPLE | 0 | 100 | 300 | 800 |
|--------|--------|--------|--------|--------|
| 1 | 0.0740 | 0.2828 | 0.5492 | 1.3282 |
| 2 | 0.0758 | 0.2428 | 0.5718 | 1.4378 |
| 3 | 0.0788 | 0.2411 | 0.5151 | 1.4405 |
| 4 | 0.0782 | 0.2625 | 0.5484 | 1.4400 |
| 5 | 0.0782 | 0.2215 | 0.4998 | 1.5807 |
| 6 | 0.0831 | 0.2604 | 0.6280 | 1.4487 |
| 7 | 0.0797 | 0.2401 | 0.5184 | 1.3854 |
| 8 | 0.0817 | 0.2434 | 0.4948 | 1.3540 |
| MEAN | 0.0787 | 0.2493 | 0.5407 | 1.4269 |
| S.D. | 0.003 | 0.017 | 0.041 | 0.072 |

### TABLE 11
### ABSORBANCE OF GLUCOSE STANDARDS (mg/dL) AT 560 nm

| SAMPLE | 0 | 100 | 300 | 800 |
|---|---|---|---|---|
| 1 | 0.0671 | 0.1731 | 0.3134 | 0.7205 |
| 2 | 0.0660 | 0.1520 | 0.3260 | 0.7828 |
| 3 | 0.0704 | 0.1512 | 0.2957 | 0.7837 |
| 4 | 0.0691 | 0.1642 | 0.3134 | 0.7825 |
| 5 | 0.0619 | 0.1431 | 0.2857 | 0.8551 |
| 6 | 0.0715 | 0.1642 | 0.3537 | 0.7884 |
| 7 | 0.0692 | 0.1522 | 0.2964 | 0.7495 |
| 8 | 0.0698 | 0.1547 | 0.2831 | 0.7338 |
| MEAN | 0.0681 | 0.1568 | 0.3084 | 0.7745 |
| S.D. | 0.003 | 0.009 | 0.022 | 0.039 |

### EXAMPLE 18

PREPARATION OF MATRICES CONTAINING CHOLESTEROL ESTER HYDROLASE AND CHOLESTEROL OXIDASE

0.374 g of 4-aminoantipyrine (available from Sigma Chemical Co., St. Louis, MO), 0.299 g of cholesterol ester hydrolase (available from Amano, a division of Mitsubishi, Troy, VA), 0.139 g of cholesterol oxidase (available from Boehringer Mannheim, Indianapolis, IN), were placed into solution with 20.4mL of 50 mM MOPSO buffer (3-[N-Morpholino]-2-hydroxypropanesulfonic acid, available from Sigma Chemical Co., St. Louis, MO) at pH 7.0.

Four types of Porex® matrices containing different amounts of detergent were tested. These matrices are available from Porex® Inc. (Fairburn, Georgia) as product numbers LC06 WW5.3, containing 0.3% of detergent WW5: LC06 WW5.2, containing 0.2% of detergent WW5; LC06 WW5.1, containing 0.1% of detergent WW5; and LC06, which contained no detergent.

The matrices were saturated with this component solution, frozen and lyophilized according to the method of the invention.

### EXAMPLE 19

The stability of the matrices of Example 6 was tested by placing the matrices in vials and incubating the vials at 45°C for 3 days without dessicant and determining the percent recovery of cholesterol ester hydrolase (CEH), cholesterol oxidase (CO) and 4-aminoantipyrine (4 AAP) ("stressed") and comparing these results to the results obtained when matrices of Example 6 were stored at 4°C under dessicant for 3 days ("control"). These results are shown in Table 4.

### TABLE 12

| MATRIX | CONDITION | PERCENT RECOVERY OF EXPECTED ACTIVITY | | |
|---|---|---|---|---|
| | | CEH | CO | 4-AAP |
| LCO6WW5.3 | CONTROL | 84% | 96% | 102% |
| | STRESSED | 1% | 0% | 111% |
| LCO6WW5.2 | CONTROL | 72% | 106% | 95% |
| | STRESSED | 48% | 1% | 91% |
| LCO6WW5.1 | CONTROL | 68% | 81% | 97% |
| | STRESSED | 43% | 4% | 97% |
| LCO6 | CONTROL | 58% | 94% | 101% |
| | STRESSED | 34% | 8% | 99% |

EXAMPLE 20

PREPARATION OF MATRICES CONTAINING CHOLESTEROL REAGENTS

The following were added to a total of 10.2 mL of 250 mM MOPSO (pH 7.0):

0.0113 g magnesium chloride (reagent grade, available from Fisher), 0.0276 g calcium chloride (reagent grade, available from Fisher), 0.5100 g lactose (available from Sigma Chemical Co., St. Louis, MO), 1.0200 g Bovine Serum Albumin (fatty acid free, available from Sigma Chemical Co., St. Louis, MO), 0.3060 g glycerol (available from Sigma Chemical Co., St. Louis, MO), 0.1868 g 4-aminoantypyrine (available from Sigma Chemical Co., St. Louis, MO), 2200 units cholesterol ester hydrolase (available from Amano, a division of Mitshubishi, Troy, VA), 260 units cholesterol oxidase (available from Boehringer Mannheim, Indianapolis, IN).

The reagent was loaded into Porex LC06 matrices having a nominal void volume of 8.5 μL according to the method of the invention. A vacuum of 84.5 kPa (25 inches of mercury) was applied to the matrices/solution mixture three times to enhance entrance of the solution into the matrices. The container then was frozen and lyophilized according to the method of the invention. Powder from the excess solution needed to assure complete frit loading was removed by shaking the lyophilized matrices on a #10 sieve for 15 minutes.

EXAMPLE 21

PREPARATION OF MATRICES CONTAINING PEROXIDASE/DCHBS REAGENTS

Matrices containing horseradish peroxidase (POD) and DCHBS were prepared as follows. 0.8064 g of DCHBS was dissolved in approximately 8.5 mL of 50 mM MOPSO at pH 7.0. The pH was adjusted with 12N HCl to pH 7.0, and 37,500 units of horseradish peroxidase (available from Amano, a division of Mitsubishi, Troy, VA) was dissolved in this solution. The final volume was adjusted to 10.2 mL with 50 mM MOPSO at pH 7.0.

1000 matrices having a nominal void volume of 8.5 μL per matrix were added to the solution. The mixture was stirred, frozen and lyophilized according to the method of the invention. Excess solution was removed by shaking the loaded matrices-on a #10 sieve for 15 minutes.

EXAMPLE 22

PREPARATION OF MATRICES CONTAINING GLYCEROL-PHOSPHATE OXIDASE AND GLYCEROL KINASE

A component solution was prepared which contained 0.0/297 g/mL of adenosine triphosphate disodium salt (ATP, available from Sigma Chem. Co., St. Louis, MO), 0.0094 g/mL of magnesium chloride (available from Sigma Chem. Co., St. Louis, MO), 0.0047 g/mL of 4-aminoantipyrine (available from Sigma Chem. Co., St. Louis, MO), 0.0500 g/mL sucrose (Sigma Chem. Co., St. Louis, MO) 0.0731 g/mL bovine serum albumin (BSA, fatty acid free, available from Sigma Chem. Co., St. Louis, MO), 0.0146 g/mL polyethylene glycol (PEG) (available from Sigma Chem. Co., St. Louis, MO), 769 U/mL glycero-3-phosphate oxidase (available from Kodak, Rochester, NY), 102540 U/mL Lipase (available from Toyo Jozo, Tokyo, Japan) and 39 U/mL of glycerokinase (available from Kodak, Rochester, NY) in 6.5 mL of 50 mM phosphate buffer at pH 7.0. Then 100 μL of freshly prepared ferrocyanide solution (1.2672 g of potassium ferrocyanide trihydrate in 100 mL of distilled water, available from Sigma Chem. Co., St. Louis, MO) was added to the mixture. A quantity of Porex® LC06 WW5.3 matrices of sintered polyethylene, having a nominal matrix volume of approximately 6.5 μL per matrix, were saturated with this component solution, frozen and lyophilized according to the method of the invention.

EXAMPLE 23

PREPARATION OF MATRICES CONTAINING PEROXIDASE AND LIPASE

A component solution was prepared which contained 0.1041 g/mL of DCHBS, 102540 U/mL of Lipase (Toyo Jozo Co., Ltd., Tokyo, Japan) and 808 U/mL of horseradish peroxidase in 6.5 mL of phosphate buffer. A quantity of Porex LC06 WW5.3 matrices of sintered polyethylene, having a nominal matrix volume of approximately 6.5 μL, were saturated with this component solution, frozen and lyophilized according to the method of the invention.

EXAMPLE 24

ASSAY FOR TRIGLYCERIDES

Matrices prepared according to Example 10 and 11 were added to a cuvette. 500 μL of Phosphate Buffer

pH 7.0, containing a detergent, Genapol (available from Hoechst, located in West Germany, and Triton® X-100 (Sigma Chem. Co., St. Louis, MO) was added to the cuvette. 6 µL of a triglyceride standard (available from Abbott Laboratories, Abbott Park, IL, as a triglyceride standard for Abbott Vision®) (without preservatives) at either 0, 63.44, 161.08 and 879.12 mg/dL was added to a cuvette and incubated. Each standard was tested six times, and absorbance readings were averaged to obtain a mean absorbance. These data are presented in Table 5.

TABLE 13
ABSORBANCE OF TRIGLYCERIDE STANDARDS (mg/dL) at 512 nm

| SAMPLE | 0 | 63.44 | 161.08 | 879.12* |
|--------|-------|-------|--------|---------|
| 1 | 0.141 | 0.437 | 0.759 | 3.142 |
| 2 | 0.136 | 0.396 | 0.837 | 3.364 |
| 3 | 0.134 | 0.395 | 0.809 | 3.364 |
| 4 | 0.112 | 0.439 | 0.849 | 3.342 |
| 5 | 0.136 | 0.427 | 0.831 | 2.814 |
| 6 | 0.132 | 0.419 | 0.823 | 3.060 |
| Mean | 1.132 | 0.419 | 0.818 | 3.194 |

*The absorbance data for the 879.12 mg/dL standard was determined by a sample dilution of 1:1 with phosphate buffer (pH 7.0) containing Geropol and Triton® X-100, and multiplying the result by a factor of two. This was necessary due to the range limits of the spectrophotometer used in the assay.

Tables 9, 10 and 11 demonstrate data obtained from absorbance readings taken of glucose standards at 0, 100, 300 and 800 mg/dl at 450 nm, 512 nm and 560 nm. Each standard was tested 8 times (N=8). The mean and standard deviation for each concentration of glucose standard are shown. The data demonstrate that little variance occurred from sample to sample, indicating the reproducibility and reliablity of the delivery system.

The data of Table 12 indicate that the recovery of cholestrol oxidase activity was greatest from the matrix LCO6, the hydrophobic matrix.

The data of Table 13 demonstrates a linear dose response for the triglycerides assay.

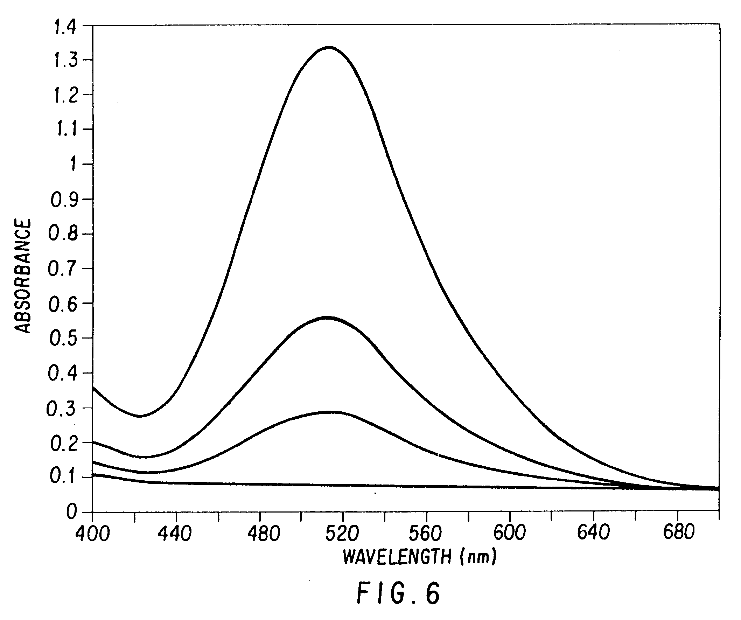

Referring to FIG. 6, the graph demonstrates the spectra obtained by different concentrations of glucose standards plotted as a function of absorbance v. wavelength. The highest peak was obtained by a glucose standard of 800 mg/dL at approximately 512 nm. The second highest peak was obtained by a glucose standard of 300 mg/dL at approximately 512 nm. The third highest peak was obtained by a glucose standard of 100 mg/dL. A flat line was obtained when a glucose standard at 0 mg/dL (distilled water) was tested.

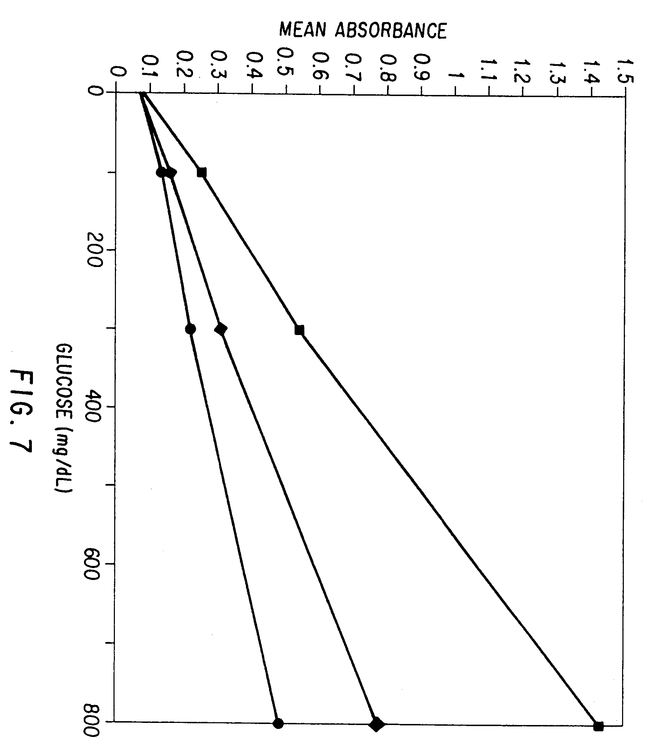

Referring to FIG. 7, the graph demonstrates the linear relationship between absorbance at a given wavelength and standard glucose concentration. It is noteworthy that separate lines were obtained at different wavelengths indicating that values can be obtained at various wavelengths along the absorbance spectra.

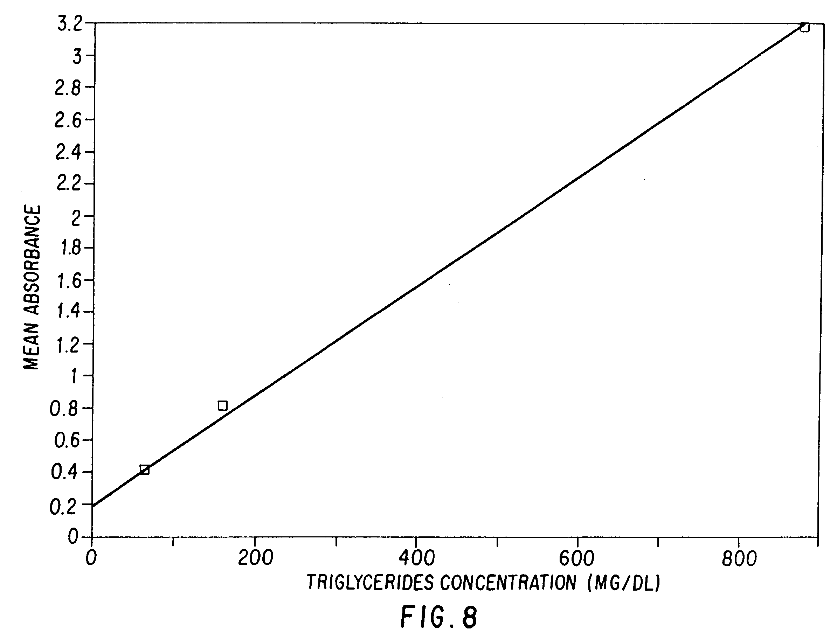

Referring to FIG. 8, the graph demonstrates the linear relationship between absorbance at 512 nm and standard triglyceride concentration.

The concepts of the present invention are applicable to various types of assays and materials other than those specifically described herein. It will be appreciated that one skilled in the art can conceive of many other assays and materials to which the present inventive concepts can be applied. The embodiments described and the alternative embodiments presented are intended as examples rather than as limitations. The description of the invention is not intended to limit the invention to the particular embodiments disclosed. Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. A device for separating plasma or serum from whole blood comprising a matrix of hydrophilic sintered porous material in which at least one red blood cell agglutinating agent has been incorporated, said matrix being characterized by a pore size selected such that individual blood cells pass through said matrix but agglutinated blood cells are retained by said matrix.

2. The device according to Claim 1, wherein said sintered porous material is selected from the group consisting of sintered glass, sintered steel, sintered ceramics, and sintered plastics.

3. The device according to Claim 1, wherein the pore size of said matrix is from about 10 μm to about 70 μm, and the quantity and type of agglutinating agents are selected such that at least 95% of the red blood cells contained within a blood sample added to the advice are retained by the matrix.

4. The device according to Claim 1, further comprising a filter means in liquid receiving relationship with said matrix wherein said filter means is capable of retaining blood cells which pass through said matrix and wherein said filter means is selected from the group consisting of derivatized or underivatized cellulose filter and porous polyethylene matrices.

5. The device according to Claim 1, further comprising a second matrix of hydrophilic sintered porous material in liquid receiving relationship with said first matrix which is capable of receiving a selected predetermined amount of plasma or serum from said first matrix.

6. The device according to Claim 1, further comprising additional matrices or filter means in liquid receiving relationship with said first matrix, wherein an analytical reagent selected for reaction with a selected component is incorporated within a member selected from the group consisting of said matrices and said filter means.

7. A method for separating plasma or serum from whole blood comprising the steps:
   (a) applying a sample of whole blood to an inlet of a device comprising a matrix of hydrophilic sintered porous material in which at least one red blood cell agglutinating agent has been incorporated, said matrix being characterized by a pore size selected such that individual blood cells pass through said matrix but agglutinated blood cells are retained by said matrix; and
   (b) collecting the plasma or serum from an outlet of the device.

8. The method according to Claim 7, wherein the pore size of said matrix and the quantity and type of agglutinating agents are selected such that at least 95% of red blood cells contained within said blood sample are retained by the matrix.

9. The method according to Claim 7, wherein said blood sample which has been applied to the inlet of said matrix is passed into a filter means in liquid receiving relationship with the outlet of said matrix and wherein said filter means functions to retain blood cells which pass through said matrix.

10. The method according to Claim 7, wherein said plasma or serum is collected by means of a second matrix of hydrophilic sintered material in liquid receiving relationship with said first matrix and wherein said plasma or serum is eluted from said second matrix by means of an elution buffer.


## Patentansprüche

1. Vorrichtung zur Trennung von Plasma oder Serum von Vollblut, die eine Matrix aus hydrophilem gesintertem porösem Material umfaßt, in das wenigstens ein rote Blutkörperchen agglutinierendes Agens eingebaut worden ist, wobei die Matrix durch eine Porengröße gekennzeichnet ist, die derart gewählt ist, daß einzelne Blutkörperchen die Matrix passieren, aber agglutinierte Blutkörperchen von dieser Matrix zurückgehalten werden.

2. Vorrichtung nach Anspruch 1, wobei das gesinterte poröse Material aus der Gruppe bestehend aus gesintertem Glas, gesintertem Stahl, gesinterter Keramik und gesintertem Kunststoff gewählt ist.

**3.** Vorrichtung nach Anspruch 1, wobei die Porengröße der Matrix von ungefähr 10 µm bis ungefähr 70 µm reicht, und wobei Anzahl und Art der agglutinierenden Agentien so gewählt sind, daß wenigstens 95% der roten Blutkörperchen, die in einer zu der Vorrichtung gegebenen Blutprobe enthalten sind, von der Matrix zurückgehalten werden.

**4.** Vorrichtung nach Anspruch 1, die ferner eine Filtervorrichtung umfaßt, die so angeordnet ist, daß sie Flüssigkeit aus der Matrix erhält, wobei die Filtervorrichtung in der Lage ist, die Blutkörperchen, die die Matrix passieren, zurückzuhalten, und wobei die Filtervorrichtung aus der Gruppe, bestehend aus derivatisierten oder underivatisierten Zellulosefilter- und porösen Polyethylenmatrizen, gewählt ist.

**5.** Vorrichtung nach Anspruch 1, die ferner eine zweite Matrix aus hydrophilem gesintertem porösem Material umfaßt, die so angeordnet ist, daß sie Flüssigkeit aus der ersten Matrix erhält, und die fähig ist, eine ausgewählte vorbestimmte Menge an Plasma oder Serum von der ersten Matrix zu erhalten.

**6.** Vorrichtung nach Anspruch 1, die ferner zusätzliche Matrizen oder Filtervorrichtungen umfaßt, die so angeordnet sind, daß sie Flüssigkeit aus der ersten Matrix erhalten, wobei ein analytisches Reagens, das für die Reaktion mit einer ausgewählten Komponente gewählt ist, in einem Bauteil eingebaut ist, das aus der Gruppe, bestehend aus den Matrizen und Filtervorrichtungen, gewählt ist.

**7.** Verfahren zur Trennung von Plasma oder Serum von Vollblut, welches folgende Schritte umfaßt:
(a) Aufgeben einer Probe Vollblut auf den Einlaß einer Vorrichtung, die eine Matrix aus hydrophilem gesintertem porösem Material umfaßt, in welchem wenigstens ein die roten Blutkörperchen agglutinierendes Agens eingebaut ist, wobei die Matrix durch eine so gewählte Porengröße gekennzeichnet ist, daß einzelne Blutkörperchen diese passieren, wobei aber agglutinierte Blutkörperchen von der Matrix zurückgehalten werden, und
(b) Sammeln des Plasma oder Serum aus einem Auslaß der Vorrichtung.

**8.** Verfahren nach Anspruch 7, wobei die Porengröße der Matrix und die Menge und Art von agglutinierendem Agens so gewählt sind, daß wenigstens 95% der in der Blutprobe enthaltenen roten Blutkörperchen von der Matrix zurückgehalten werden.

**9.** Verfahren nach Anspruch 7, wobei die Blutprobe, die auf den Einlaß der Matrix aufgegeben worden ist, eine Filtervorrichtung passiert, die so angeordnet ist, daß sie Flüssigkeit aus dem Auslaß der Matrix erhält, und wobei die Filtervorrichtung derart arbeitet, daß Blutkörperchen, die die Matrix passieren, zurückgehalten werden.

**10.** Verfahren nach Anspruch 7, wobei das Plasma oder Serum mittels einer zweiten Matrix aus hydrophilem gesintertem Material gesammelt wird, die so angeordnet ist, daß sie die Flüssigkeit von der ersten Matrix erhält, und wobei das Plasma oder Serum von der zweiten Matrix mittels eines gepufferten Laufmittels eluiert wird.

**Revendications**

**1.** Dispositif de séparation du plasma et du sérum à partir de sang entier comprenant une matrice en matériau poreux hydrophile fritté dans lequel au moins un agent agglutinant des hématies a été incorporé, ladite matrice étant caractérisée par une dimension de pores choisie de sorte que les cellules sanguines individuelles passent par ladite matrice, mais que les cellules sanguines agglutinées sont retenues sur ladite matrice.

**2.** Dispositif selon la revendication 1, dans lequel on choisit ledit matériau poreux fritté dans le groupe constitué par le verre fritté, l'acier fritté, la céramique frittée et le plastique fritté.

**3.** Dispositif selon la revendication 1, dans lequel la dimension de pores de ladite matrice est comprise entre environ 10 µm et environ 70 µm et dans lequel on choisit la quantité et le type des agents agglutinants de sorte qu'au moins 95 % des hématies contenues dans un échantillon sanguin ajouté au dispositif sont retenus sur la matrice.

**4.** Dispositif selon la revendication 1, comprenant de plus un élément filtrant en relation de réception de liquide avec ladite matrice, dans lequel ledit élément filtrant est capable de retenir les cellules sanguines qui passent par ladite matrice et dans lequel on choisit ledit élément filtrant dans le groupe constitué par un filtre en cellulose dérivée ou non dérivée et des matrices poreuses en polyéthylène.

5. Dispositif selon la revendication 1, comprenant de plus une deuxième matrice en matériau poreux hydrophile fritté en relation de réception de liquide avec ladite première matrice qui est capable de recevoir une quantité choisie prédéterminée de plasma ou de sérum à partir de ladite première matrice.

6. Dispositif selon la revendication 1, comprenant de plus des matrices ou des éléments filtrants supplémentaires en relation de réception de liquide avec ladite première matrice, dans lesquels on incorpore un réactif d'analyse, choisi pour une réaction avec un composant sélectionné, dans un élément choisi dans le groupe constitué par lesdites matrices et lesdits appareils filtrants.

7. Procédé de séparation du plasma et du sérum à partir de sang entier comprenant les étapes consistant à :
   (a) appliquer un échantillon de sang entier à l'entrée d'un dispositif comprenant une matrice en matériau poreux hydrophile fritté dans lequel au moins un agent agglutinant des hématies a été incorporé, ladite matrice étant caractérisée par une dimension de pores choisie de sorte que les cellules sanguines individuelles passent par ladite matrice, mais que les cellules sanguines agglutinées sont retenues par ladite matrice et
   (b) recueillir le plasma et le sérum à partir d'une sortie du dispositif.

8. Procédé selon la revendication 7, dans lequel on choisit la dimension de pores de ladite matrice, ainsi que la quantité et le type des agents agglutinants, de sorte qu'au moins 95 % des hématies contenues dans ledit échantillon sanguin sont retenus par la matrice.

9. Procédé selon la revendication 7, dans lequel on fait passer ledit échantillon sanguin, qui a été appliqué à l'entrée de ladite matrice, dans un élément filtrant en relation de réception de liquide avec la sortie de ladite matrice et dans lequel ledit élément filtrant opère pour retenir les cellules sanguines qui passent par la matrice.

10. Procédé selon la revendication 7, dans lequel on recueille ledit plasma ou ledit sérum au moyen d'une deuxième matrice en matériau hydrophile fritté, en relation de réception de liquide avec ladite première matrice, et dans lequel on élue ledit plasma ou ledit sérum à partir de ladite deuxième matrice au moyen d'un tampon d'élution.

FIG. 1

FIG. 2

FIG. 3

FIG. 4

FIG. 5

ABSORBANCE

FIG. 6

WAVELENGTH (nm)

FIG. 7

MEAN ABSORBANCE

GLUCOSE (mg/dL)

FIG. 8